# EUROPEAN PATENT APPLICATION

(11) **EP 2 093 218 A1**
(43) Date of publication of application: **26.08.2009**
(21) Application number: 08101884.8
(22) Date of filing: 22.02.2008
(51) Int. Cl.: C07D 233/32, A61K 31/4166

(54) **Arylalkyl substituted imidazolidinones**

(71) Applicant: Fariello, Ruggero, 21016 Luino (VA) (IT)
(72) Inventor: Cattaneo, Carlo, 21040, GERENZANO (IT); Fariello, Ruggero, 21040, GERENZANO (IT); Maj, Roberto, 21040, GERENZANO (IT)
(74) Representative: Gerli, Paolo

(57) **Abstract**

New compounds of formula (I), 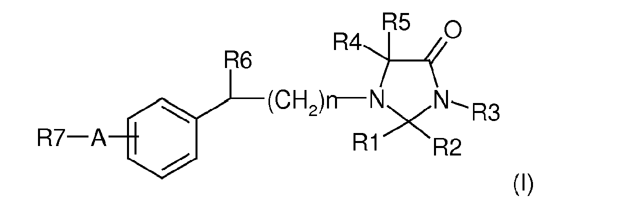 wherein: R1, R2, R3, R4 and R5 are, independently from each other, hydrogen; C₁₋₆alkyl; aryl; heteroaryl and perhaloalkyl, and wherein at least one of R1 and R2 is different from hydrogen; n is 0 or 1; R6 is hydrogen or C₁₋₆alkyl; A is a group - (CH₂)ₘ-, -(CH₂)ₚ-X- or -X-(CH₂)ₚ- where m = 1-4, p = 0-4, X is -O-, -S- or -NR8-, where R8 is hydrogen or C₁₋₄alkyl; R7 is C₁₋₆alkyl; C₃₋₈cycloalkyl; aryl; heteroaryl.

## Description

### FIELD OF THE INVENTION

The present invention relates to new arylalkyl substituted imidazolidinones, a process for their preparation, pharmaceutical compositions containing them, and the use of these compounds in medicine and biochemistry.

### BACKGROUND OF THE INVENTION

Epilepsy is one of the most prevalent neurological syndromes, currently affecting about 50 million people world-wide. The term epilepsy describes a group of brain disorders sharing seizures as a common behavioural manifestation. Classical antiepileptic drugs (AEDs), such as phenobarbital, phenytoin (PHT), carbamazepine (CBZ) and valproic acid (VPA) do not produce complete or satisfactory seizure control in approximately 30% of the treated patients. The response rate varies in relation to seizure type, from excellent (for example, in typical absence seizures) to intermediate (complex partial seizures), to very poor (atonic and tonic seizures associated with the Lennox-Gastaut syndrome).

The search for new AEDs is not restricted to the treatment of unresponsive patients, but also to the reduction of side effects of classical AEDs ; in fact all these drugs exhibit several undesirable effects, such as: 1) adverse CNS effects i.e. ataxia, sedation, nystagmus, headache, extrapyramidal effects, slowing of cognition, mental confusion, encephalopathy; 2) teratogenicity; 3) significant potential for idiosyncratic reactions i.e.: VPA for acute hepatic necrosis; CBZ for myelosuppression; PHT and ethosuximide for lymphoma, lupus; 4) unfavourable kinetics; 5) drug to drug interaction, resulting from enzyme induction or inhibition.

Therefore, notwithstanding the beneficial effects of current drugs, the neurologists still need new AEDs with potent anticonvulsant effect and endowed in particular with low toxicity. A new agent possessing these characteristics has a good chance to become a first line treatment.

Some imidazolidin-4-one with certain substitution patterns have been described as synthetic intermediates in the preparation of proteinogenic aminoacids (EP 542009, EP 237630) or antibiotics (J.Org.Chem., 1992, 57(1), 10-11), others are known as herbicides (DE 3408403) or cardioprotective agents (DE 2151216).

### SUMMARY OF THE INVENTION

We have now discovered new compounds of formula (I), 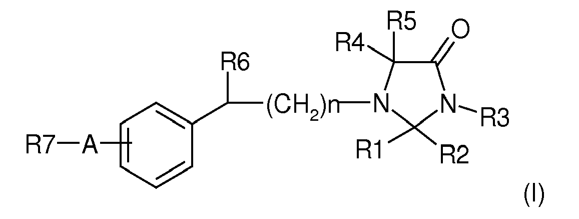 wherein:
**R1, R2, R3, R4** and **R5** are, independently from each other, hydrogen, C₁₋₆alkyl, aryl, heteroaryl or perhaloalkyl, and wherein at least one of R1 and R2 is different from hydrogen;
*n* is 0 or 1;
**R6** is hydrogen or C₁₋₆alkyl;
**A** is a group -(CH₂)ₘ-, -(CH₂)ₚ-X- or -X-(CH₂)ₚ- where *m* = 1-4, *p* = 0-4, X is -O-, - S- or -NR8- where R8 is hydrogen or C₁₋₄alkyl;
**R7** is C₁₋₆alkyl, C₃₋₈cycloalkyl, aryl or heteroaryl
and where each alkyl, cycloalkyl, aryl and heteroaryl group may be substituted by halogen, hydroxy, C₁₋₆alkoxy, amino, (C₁₋₆alkyl)amino, di(C₁₋₆alkyl)amino, perhaloalkyl.

These compounds are useful in therapy as sodium channel inhibitors, for the treatment/prevention of seizures and other diseases.

### DETAILED DESCRIPTION OF THE INVENTION

In the present invention all "alkyl" groups can be indifferently linear or branched; said alkyl groups are preferably C₁₋₄alkyl groups, more preferably methyl or ethyl.

All "aryl" groups are preferably C₅₋₁₀aryl groups, in particular phenyl and naphthyl.

The term "halogen" includes the fluorine, chlorine, bromine and iodine groups, preferably chlorine and fluorine.

The term "heteroaryl" includes saturated or unsaturated heterocyclic rings containing up to 3 heteroatoms chosen among O, S and N.

Preferably, R1 and R2 are chosen among hydrogen, C₁₋₄alkyl, aryl; more preferably, R1 and R2 are C₁₋₄alkyl; even more preferably, R1 and R2 are methyl.

Preferably, R3 is hydrogen or C₁₋₄alkyl; more preferably, R3 is hydrogen or methyl

Preferably, R4 and R5 are hydrogen or C₁₋₄alkyl; more preferably, R4 is hydrogen and R5 is methyl.

Preferably, *n* is 0

Preferably, R6 is hydrogen or C₁₋₂alkyl; more preferably, R6 is hydrogen.

Preferably, A is -(CH₂)ₚ-X- or -X-(CH₂)ₚ-, with *p* = 0-2; more preferably, A is - CH₂O-.

Preferably, R7 is aryl; more preferably, R7 is aryl substituted by halogen; even more preferably, R7 is 2-fluorophenyl or 3-fluorophenyl.

Specific compounds of formula (I) according to the present invention (of which each also comprises the corresponding salts such as e.g. hydrochloride, trifluoroacetate or methanesulfonate, and hydrates) are:
(S)-(-)-1-[4-(3-Fluoro-benzyloxy)-benzyl]-2,2,5-trimethyl-imidazolidin-4-one
(S)-(-)-1-[4-(2-Fluoro-benzyloxy)-benzyl]-2,2,5-trimethyl-imidazolidin-4-one.

The compounds of formula (I) can exhibit stereoisomerism because of the presence of chiral atoms and/or multiple bonds. The present invention therefore extends to stereoisomers of the compounds of formula (I), including racemates and mixtures where the enantiomers are present in any proportions, enantiomers, diastereoisomers and geometric isomers.

The subject invention also includes isotopically-labelled compounds, which are identical to those recited in formula (I) and following, but for the fact that one or more atoms are replaced by an atom having an atomic mass or mass number different from the atomic mass or mass number usually found in nature. Examples of isotopes that can be incorporated into compounds of the invention and pharmaceutically acceptable salts thereof include isotopes of hydrogen, carbon, nitrogen, oxygen, phosphorous, sulphur, fluorine, iodine, and chlorine, such as ²H, ³H, ¹¹C, ¹³C, ¹⁴C, ¹⁵N, ¹⁷O, ¹⁸O, ³¹P, ³²P, ³⁵S, ¹⁸F, ³⁶Cl, ¹²³I and ¹²⁵I.

Compounds of the present invention and pharmaceutically acceptable salts or hydrates of said compounds that contain the aforementioned isotopes and/or other isotopes of other atoms are within the scope of the present invention. They are useful as diagnostic tools as tracers or markers of neurological functions and dysfunctions correlated to the involvement of voltage-sensitive ion channels. Isotopically-labelled compounds of the present invention, for example those into which radioactive isotopes such as ³H, ¹⁴C are incorporated, are useful in drug and/or substrate tissue distribution assays. Tritiated, i.e., ³H, and carbon-14, i.e., ¹⁴C isotopes are particularly preferred for their ease of preparation and detectability. ¹¹C and ¹⁸F isotopes are particularly useful in PET (positron emission tomography), and ¹²⁵I isotopes are particularly useful in SPECT (single photon emission computerized tomography), all useful in brain imaging. Further, substitution with heavier isotopes such as deuterium, i.e., ²H, can afford certain therapeutic advantages resulting from greater metabolic stability, for example increased *in vivo* half-life or reduced dosage requirements and, hence, may be preferred in some circumstances. Isotopically labelled compounds of formula (I) and following of this invention can generally be prepared by carrying out the procedures disclosed in the Schemes and/or in the Examples below, by substituting a readily available isotopically labelled reagent for a non-isotopically labelled reagent.

The present invention also provides processes for preparing the compounds of formula (I).

Compounds of general formula (I) can be prepared as described in Scheme 1, reacting a compound of formula (II) with a compound of formula (III), where R1, R2, R3, R4, R5, R6, R7, *n* and A have the meanings given for formula (I) and W is a suitable leaving group, such as e.g. chlorine, bromine, iodine, methanesulfonate or p-toluenesulfonate.

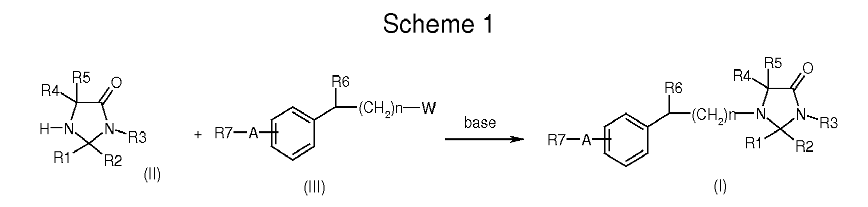

In a typical procedure, a compound of formula (III) is dissolved in a suitable solvent, in particular an aprotic dipolar solvent, for example acetonitrile, and then reacted with a compound of formula (II) in the presence of a base, such as e.g. potassium carbonate, at a temperature between 0°C and 80°C, for a period of time between 1 and 24 h. If necessary, any residual solid may be filtered off and the solvent is removed by evaporation. The crude product may be purified, if necessary, by conventional purification methods such as flash chromatography, trituration, crystallization or preparative HPLC.

Compounds of general formula (III) are either known or commercially available, or may be prepared as described in reference texts of synthetic methodology, such as March's Advanced Organic Chemistry, Sixth Edition (2007), Wiley Interscience*.* Compounds of formula (II) can be prepared according to Scheme 2, reacting a compound of formula (IV) with a compound of formula (V), where R1, R2, R3, R4 and R5 have the meanings given for formula (I).

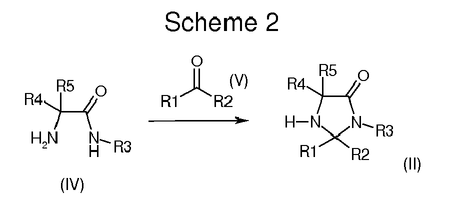

In a typical procedure, a compound of formula (IV) is dissolved in a suitable solvent, in particular an alcoholic solvent, for example, n-butanol, and reacted with a compound of formula (V); alternatively, the compound of formula (V) itself may be used as solvent or co-solvent; molecular sieves may be added to the reaction mixture to facilitate the reaction. The reaction mixture is stirred for a period of time between 1 and 48 h, at a temperature between 20°C and the reflux temperature of the chosen solvent, then the residual solids are filtered off and the solvent is removed by evaporation. The crude product may be purified, if necessary, by conventional purification methods such as flash chromatography, trituration, crystallization or preparative HPLC.

Compounds of general formula (IV) and (V) are either known or commercially available, or may be prepared as described in reference texts of synthetic methodology, such as March's Advanced Organic Chemistry, Sixth Edition (2007), Wiley Interscience*.*

Alternatively, compounds of formula (I) may be prepared as described in Scheme 3, reacting a compound of formula (VI) with a compound of formula (V), where R1, R2, R3, R4, R5, R6, R7, n and A have the meanings given for formula (I), under the conditions described above for the preparation of compounds of formula (II).

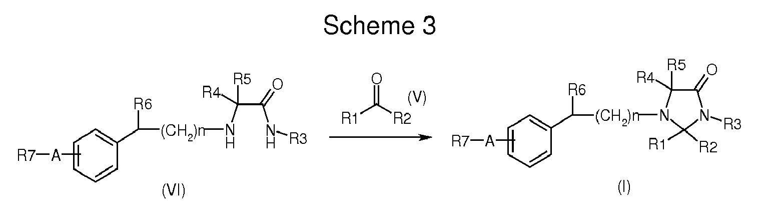

Compounds of formula (VI) may in turn be prepared according to Scheme 4, reacting a compound of formula (III) with a compound of formula (IV), where R3, R4, R5, R6, R7, n and A have the meanings given for formula (I) and W is as defined above, under the conditions described above for the preparation of compounds of formula (I).

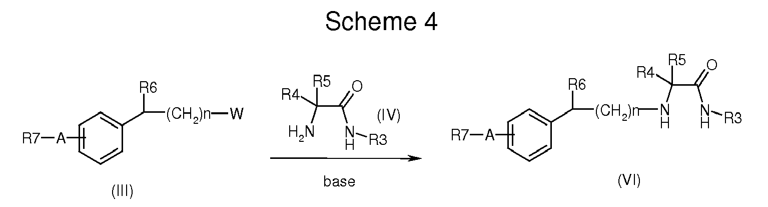

Alternatively, compounds of formula (I) can be obtained reacting compounds of formula (VII) or (VIII) respectively, where R6, A and R7 have the meaning given for formula (I), 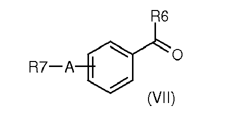 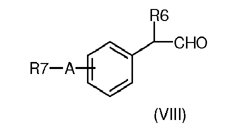 with a compound of formula (II) above, thus obtaining compounds of formula (I) in which n is 0 or 1 respectively, under reductive amination conditions well known in literature, such as e.g. those described in Lane, Synthesis, 135, 1975, employing sodium cyanoborohydride in methanol, or in Mattson, J. Org. Chem. 55, 2552, 1990, employing titanium(IV)isopropoxide and sodium cyanoborohydride.

Compounds of formula (VII) and (VIII) are either known or commercially available, or may be prepared as described in reference texts of synthetic methodology, such as March's Advanced Organic Chemistry, Sixth Edition (2007), Wily Interscience

The compounds of formula (I) are sodium channel inhibitors, which makes them useful in the treatment of a number of diseases. In particular, they are active on the central nervous system (CNS) as demonstrated in the experimental part by their activity, for example, on maximal electroshock test and chemically-induced seizures, and can be used in therapy in the treatment of epilepsy, in the treatment or prevention of depression and bipolar disorders, anxiety and schizophrenia, as antispastic and/or hypnotic agents. The compounds of the invention are also useful in the treatment or prevention of cognitive impairment including, but not limited to, amnestic disorders, memory loss and cognitive disorders, dementia as such, e.g. vascular dementia and dementia associated with AIDS; they are further effective in treating or preventing motor damage and neurodegeneration due to Alzheimer's disease; senile dementia, Parkinson's disease, Restless Legs Syndrome, Lewy-Body disease, disorders due to defective motor coordination or other neurodegenerative pathologies. The compounds of the invention are also useful as neuroprotectant in all those pathological conditions in which nervous cells (neurons) and fibers (nerves), degenerate due to whatsoever cause. The invention therefore includes the use of said compounds of formula (I) in the preparation of medicaments for treating the aforesaid diseases, as well as a method to the same disease by administering a pharmaceutically effective dose of a compound of formula (I) to a patient in need thereof.

According to the present invention, compounds of formula (I) may be administered as such or in association with any other active principle useful for the treatment or prevention of the above mentioned diseases. Non-limiting examples of other active principles to be used in association with compounds of the invention are e.g. L-Dopa, dopamine agonists (e.g. ropinirole or pramipexole) and catechol-O-methyl transferase (COMT) inhibitors (e.g. entacapone or tolcapone) for the treatment of Parkinson's disease or restless legs syndrome; acetylcholinesterase inhibitors (e.g. donepezil or galantamine) or NMDA-receptor antagonists (e.g. memantine) for the treatment of Alzheimer's disease and other neurodegenerative diseases; lithium, Selective Serotonin Reuptake Inhibitors (SSRIs) (e.g. paroxetine, fluoxetine, citalopram or escitalopram) and Selective Noradrenaline Reuptake Inhibitors (SNRI) (e.g. reboxetine) for the treatment of depression and bipolar disorders.

The compounds of formula (I) can be prepared in the forms of salts or hydrates. Suitable salts are pharmaceutically acceptable salts. Suitable hydrates are pharmaceutically acceptable hydrates.

The therapeutic regimen for the different clinical syndromes must be adapted to the type and seriousness of the pathology or pathologies to be treated, taking into account also the route of administration, the form in which the compound is administered and the age, weight and conditions of the subject involved. The compounds of the invention can be administered at doses ranging e.g. from about 10 to about 1500 mg/day.

The invention encompasses pharmaceutical compositions of compounds of formula (I) useful for the above mentioned treatments. The amounts of the active principle, expressed in mg/day, are those cited above.

The compounds of the invention may be pharmaceutically formulated according to known methodologies. The various pharmaceutical compositions may be selected according to the needs of the treatment.

Such compositions can be prepared by mixing and can be suitably adapted for oral or parenteral administration, and as such, can be administered in the form of tablets, capsules, oral preparations, powders, granules, pellets, liquid solutions for injection or infusion, suspensions or suppositories.

Tablets and capsules for oral administration are usually supplied in dosage units and may contain conventional excipients such as binders, fillers, diluents, tabletting agents, lubricants, detergents, disintegrants, colorants, flavors and wetting agents. Tablets may be coated in accordance to methods well known in the art.

Suitable fillers include for example cellulose, mannitol, lactose and similar agents. Suitable disintegrants include starch, polyvinylpyrrolidone and starch derivatives such as sodium starch glycolate. Suitable lubricants include, for example, magnesium stearate. Suitable wetting agents include for example sodium lauryl sulfate.

These solid oral compositions can be prepared with conventional mixing, filling or tabletting methods. The mixing operations can be repeated to disperse the active agent in compositions containing large quantities of fillers. These operations are conventional.

The oral liquid compositions can be provided in the form of, for example, aqueous or oily suspensions, solutions, emulsions, syrups or elixirs or in the form of a dry product to be reconstituted with water or with a suitable liquid carrier at the time of use. The liquid compositions can contain conventional additives such as suspending agents, for example sorbitol, syrup, methylcellulose, gelatin, hydroxyethylcellulose, carboxymethylcellulose, aluminium stearate gel or hydrogenated edible fats, emulsifying agents, for example lecithin, sorbitan monooleate, or acacia; non aqueous carriers (which can include edible oil) for example almond oil, fractionated coconut oil, oily esters such a glycerin esters, propylene glycol or ethyl alcohol; preservatives, for example methyl or propyl p-hydroxybenzoate or sorbic acid and if desired, conventional flavours or colorants.

Oral formulations also include conventional sustained release formulations, such as tablets or granules with enteric coating.

For parenteral administration, fluid dosage units can be prepared containing the active compounds and a sterile carrier. The active compounds, depending on the carrier and concentration, can be suspended or dissolved. The parenteral solutions are normally prepared by dissolving the compound in a carrier and sterilizing by filtration, before filling suitable vials or ampoules and sealing. Adjuvants such as local anaesthetics, preservatives and buffering agents can be advantageously dissolved in the carrier. In order to increase stability, the composition can be frozen after filling the vial and the water removed under vacuum. The parenteral suspensions are prepared essentially in the same way, with the difference that the active compounds can be suspended rather than dissolved in the carrier, and can be sterilized by exposure to ethylene oxide prior to being suspended in the sterile carrier. A surfactant or humectant can be advantageously included to facilitate uniform distribution of the compound of the invention.

A further method of administration for the compound of the invention refers to a topic treatment. Topic formulations may contain for example ointments, creams, lotions, gels, solutions, pastes and/or may contain liposomes, micelles and/or microspheres.

A further method of administration for the compounds of the invention is transdermal delivery. Typical transdermal formulations include conventional aqueous and non-aqueous vectors, such as creams, oil, lotions or pasted or may be in the form of membranes or medicated patches.

As is common practice, the compositions are normally accompanied by written or printed instructions, for use in the treatment concerned.

The compounds of formula (I) can also be used as biochemical reagents, e.g. for *in-vitro* or *in-vivo* testing, useful whenever the inhibition of sodium channels is required; they can also be used as reagents inhibiting of the various pathological occurrencies described above, e.g. seizures, in suitable *in vitro* or *in-vivo* models.

Examples of the present invention are provided in what follows, purely for illustrative and non-limiting purposes.

### EXPERIMENTAL PART

### Chemistry

### Example 1

### (S)-(-)-1-[4-(3-Fluoro-benzyloxy)-benzyl]-2,2,5-trimethyl-imidazolidin-4-one

### Step 1

L-alaninamide hydrochloride (5.4 g, 43.35 mmol) was dissolved in 300 mL of acetone and 230 mL of n-butanol; 4Å molecular sieves (5 g) and triethylamine (6 mL, 43.35 mmol) were added and the reaction mixture was heated at reflux for 32 hours, then let to cool to room temperature and filtered. The solids were discarded and the mother liquors were evaporated to dryness, obtaining 5.6 g of (S)-2,2,5-trimethyl-imidazolidin-4-one as a waxy red solid used without further purification in step 4.

### Step 2

A solution of 4-hydroxybenzylalcohol (20 g, 161 mmol) in DMF (160 mL) stirred at room temperature was added with K₂CO₃ (44.5 g, 322 mmol) and 3-fluorobenzyl chloride (21 mL, 176 mmol); the reaction mixture was heated at reflux for 3 hours and then let to cool to room temperature. The reaction mixture was added dropwise to demineralized water (1200 mL) and the resulting solid was collected by filtration on a Buckner funnel and dried at 50°C under reduced pressure to obtain 36.5 g of 4-(3-fluoro-benzyloxy)benzyl alcohol as a pink solid, used without further purification in step 3.

### Step 3

A solution of 4-(3-fluoro-benzyloxy)benzyl alcohol (crude compound from step 2, 15 g, 64.6 mmol) in dichloromethane (200 mL) under argon atmosphere was cooled to 5°C and added with CBr₄ (25.8 g, 77.8 mmol) and then with triphenylphosphine (18.6 g, 70.9 mmol) portionwise. The reaction mixture was stirred for 2 hours and then let to warm up to room temperature and evaporated to dryness. The crude compound was partially purified by chromatography on silica gel, eluting with dichloromethane, to yield 21 g of 4-(3-fluoro-benzyloxy)benzyl bromide as a pale pink solid which was used without further purification in step 4.

### Step 4

A solution of 4-(3-fluoro-benzyloxy)benzyl bromide (compound from step 3, 21 g, 71 mmol) in acetonitrile (200 mL) was added with (S)-2,2,5-trimethyl-imidazolidin-4-one (compound from step 1, 9.1 g, 71 mmol) and K₂CO₃ (4.9 g, 35.5 mmol); the reaction mixture was stirred at room temperature for 12 hours and then the residual solid filtered off and the mother liquors evaporated to dryness. The crude red oil was purified by chromatography on silica gel, eluting with a 1:1 mixture hexane/ethyl acetate, yielding 3.1 g of the title compound as a pale yellow solid.
[α]_{D}²⁵ = -17.50 (c = 1, MeOH)
¹H NMR (300 MHz, CDCl₃, δ ppm): 7.33-7.41 (m, 1H), 7.31 (m, 2H), 7.13-7.24 (m, 2H), 6.97-7.09 (m, 1H), 6.92 (m, 2H), 5.99 (br. s, 1H), 5.07 (s, 2H), 3.87 (d, 1H), 3.60 (d, 1H), 3.32 (q, 1H), 1.37 (s, 3H), 1.29 (s, 3H), 1.08 (d, 3H).
MS (ESI Pos, 3.2KV, 25V, 350°C): 343.21 (MH⁺).

### Example 2

### (S)-(-)-1-[4-(2-Fluoro-benzyloxy)-benzyl]-2,2,5-trimethyl-imidazolidin-4-one

### Step 1

A solution of 4-hydroxybenzyl alcohol (3 g, 24.2 mmol) in DMF (24 mL) stirred at room temperature was added with K₂CO₃ (6.7 g, 48.4 mmol) and 2-fluorobenzyl chloride (3.3 mL, 27.8 mmol); the reaction mixture was heated at reflux for 3 hours and then let to cool to room temperature. The reaction mixture was added dropwise to demineralised water (200 mL) and the resulting solid was collected by filtration on a Buckner funnel and dried at 50°C under reduced pressure to obtain 4.8 g of 4-(2-fluoro-benzyloxy)benzyl alcohol as a pink solid, used without further purification in step 2.

### Step 2

A solution of 4-(2-fluoro-benzyloxy)benzyl alcohol (crude compound from step 1, 2 g, 8.61 mmol) in dichloromethane (30 mL) under argon atmosphere was cooled to 5°C and added with CBr₄ (3.72 g, 11.2 mmol) and then with triphenylphosphine (2.48 g, 9.47 mmol) at portions. The reaction mixture was stirred for 2 hours and then let to warm to room temperature and evaporated to dryness. The crude was partially purified by chromatography on silica gel, eluting with dichloromethane, to give 3.5 g of 4-(2-fluoro-benzyloxy)benzyl bromide as a pink solid which was used in step 3 without further purification.

### Step 3

A solution of 4-(2-fluoro-benzyloxy)benzyl bromide (compound from step 2, 3.5 g, 11.9 mmol) in acetonitrile (50 mL) was added with (S)-2,2,5-trimethyl-imidazolidin-4-one (compound from step 1 of Example 1, 1.52 g, 11.9 mmol) and K₂CO₃ (830 mg, 6 mmol); the reaction mixture was stirred at room temperature for 12 hours and then the residual solid filtered off and the mother liquors evaporated to dryness. The crude red oil was purified by chromatography on silica gel, eluting with a 1:1 mixture hexane/ethyl acetate, to yield 126 mg of the title compound as a pink solid.
[α]_{D}²⁵ = -17.30 (c = 0.2, MeOH)
¹H NMR (300 MHz, CDCl₃, δ ppm): 7.53 (ddd, 1H), 7.29-7.39 (m, 3H), 7.18 (ddd, 1H), 7.10 (ddd, 1H), 6.95 (m, 2H), 5.85 (br. s, H), 5.15 (s, 2H), 3.87 (d, 1H), 3.61 (d, 1H), 3.32 (q, 1H), 1.37 (s, 3H), 1.29 (s, 3H), 1.08 (d, 3H).

MS (ESI Pos, 3.2KV, 25V, 350°C): 343.14 (MH⁺).

### In vitro tests

The compounds of the invention were assayed for their affinity at sodium channels according to Catterall WA et al. J. Biol. Chem. 1981, 256, 8922-8927. Compound of Example 1 was found to significantly inhibit the binding of [³H]-batrachotoxin at site 2 of rat brain sodium channels (83% inhibition @ 30 uM).

### Pharmacological methods

### 1. Maximal Electroshock Test (MES)

MES seizures were induced by electroconvulsometer (ECT Unit Ugo Basile, Italy). Mice received an electroshock (50 mA at 60 Hz for 0.2 s duration) through intra-corneal electrodes. This current intensity elicited complete tonic extension of the hindlimbs in control mice. The stimulus was applied 30 min or 60 min after intraperitoneal (i.p.) or oral (p.o.) administration of the test compound, respectively. To evaluate the drug effect on the seizure severity, time of Straightening Reflex Loss and the presence of tonic hindlimb extension were selected as the parameters.

### 2. Chemically induced seizures

Seizure-inducer pentylenetetrazole (PTZ) (90 mg/kg i.p.) was administered to mice 30 min after i.p. administration of the test compound.

### Results

### 1. Maximal Electroshock Test (MES)

Results obtained with compounds of the invention are reported in Table 1. Compound of Example 1 either after i.p. (10 mg/kg) or oral (30 mg/kg) administration showed significant anticonvulsant activity, as evidenced by the increased time of straightening reflex loss and the protection from tonic extension with respect to control animals.

**Table 1**

| **Anticonvulsant activity of compounds of the invention in comparison with diazepam in mice assessed by the MES test** | | |
|---|---|---|
| *Treatment* | *Time of Straightening Reflex Loss (s)* | *MES % Protection from Tonic Extension* |
| SALINE (i.p.) | 36.3 ± 5.9 | 0 |
| Example 1 (10 mg/kg i.p.) | 66.8 ± 9.3* | 50* |
| CMC (p.o.) | 31.4 ± 6.2 | 0 |
| Example 1 (30 mg kg⁻¹ p.o.) | 56.8 ± 6.3* | 30 |
| *P< 0.01; Each value represents the mean of 20 mice, except saline (40 mice) | | |

### 2. Chemically induced seizures

Results obtained with compounds of the invention are reported in Table 2. Compound of Example 1 at the doses of 10 and 30 mg/kg i.p. was able to protect the animals from PTZ-induced convulsions. At the highest dose, protection was almost complete.

**Table 2**

| **Anticonvulsant activity of compounds of the invention in PTZ-treated mice** | |
|---|---|
| *Treatment* | *% Protection on PTZ induced convulsions* |
| Example 1 (3 mg/kg i.p.) | 0 |
| Example 1 (10 mg/kg i.p.) | 40 |
| Example 1 (30 mg/kg i.p.) | 90 |
| Each value represents the mean of 20 mice. | |

## Claims

1. Compounds of formula (I), 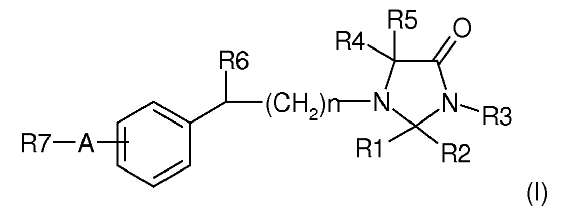 wherein:
**R1, R2, R3, R4** and **R5** are, independently from each other, hydrogen, C₁₋₆alkyl, aryl, heteroaryl or perhaloalkyl, and wherein at least one of R1 and R2 is different from hydrogen;
*n* is 0 or 1;
**R6** is hydrogen or C₁₋₆alkyl;
**A** is a group -(CH₂)ₘ-, -(CH₂)ₚ-X- or -X-(CH₂)ₚ- where *m* = 1-4, *p* = 0-4, X is -O-, - S- or -NR8- where R8 is hydrogen or C₁₋₄alkyl;
**R7** is C₁₋₆alkyl, C₃₋₈cycloalkyl, aryl or heteroaryl.

2. Compounds according to claim 1, wherein:
R1 and R2 are independently hydrogen, C₁₋₄alkyl, or aryl; R3 is hydrogen or C₁₋₄alkyl; R4 and R5 are independently hydrogen or C₁₋₄alkyl; n is 0; R6 is hydrogen or C₁₋₂alkyl; A is -(CH₂)ₚ-X- or -X-(CH₂)ₚ-, with *p* = 0-2; R7 is aryl.

3. Compounds according to claim 2, wherein:
R1 and R2 are independently C₁₋₄alkyl; R3 is hydrogen or methyl, R4 is hydrogen, R5 is methyl, *n* is 0, R6 is hydrogen, A is -CH₂O-, R7 is aryl substituted by halogen.

4. Compounds according to claim 3, wherein:
R1 and R2 are methyl, R3 is hydrogen or methyl, R4 is hydrogen and R5 is methyl, n is 0, R6 is hydrogen, A is -CH₂O-, R7 is 2-fluorophenyl or 3-fluorophenyl.

5. Compounds according to claim 4, selected from:
(S)-(-)-1-[4-(3-Fluoro-benzyloxy)-benzyl]-2,2,5-trimethyl-imidazolidin-4-one
(S)-(-)-1-[4-(2-Fluoro-benzyloxy)-benzyl]-2,2,5-trimethyl-imidazolidin-4-one

6. Process to prepare the compounds of formula (I), 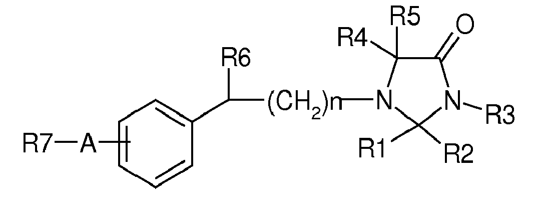 wherein:
**R1, R2, R3, R4** and **R5** are, independently from each other, hydrogen, C₁₋₆alkyl, aryl, heteroaryl or perhaloalkyl, and wherein at least one of R1 and R2 is different from hydrogen;
*n* is 0 or 1;
**R6** is hydrogen or C₁₋₆alkyl;
**A** is a group -(CH₂)ₘ-, -(CH₂)ₚ-X- or -X-(CH₂)ₚ- where m = 1-4, *p* = 0-4, X is -O-, - S- or -NR8- where R8 is hydrogen or C₁₋₄alkyl;
**R7** is C₁₋₆alkyl, C₃₋₈cycloalkyl, aryl or heteroaryl,
comprising the step of reacting a compound (IX) 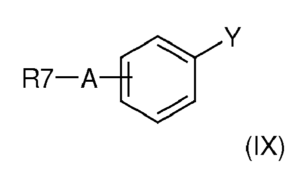 wherein **Y** is -CHR6-(CH₂)ₙ-W with W being a suitable leaving group, -CHR6-CHO, or C(R6)=O, with a compound (II), or an open-ring precursor thereof.

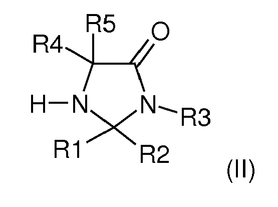

7. Process according to claim 6, wherein Y is -CHR6-(CH₂)ₙ-W, in which the reaction of (IX) with (II) takes place in an aprotic dipolar solvent in presence of a base, at temperature comprised between 0 and 80°C, for a time comprised between 1 h and 24 h.

8. Process according to claims 6-7, wherein W is Cl, Br, I, methanesulfonate or p-toluenesulfonate.

9. Process according to claim 6, wherein Y is -CHR6-CHO, or C(R6)=O, in which the reaction of (IX) with (II) takes place under reductive amination conditions.

10. Process according to claim 6-9, wherein the open ring precursor has structure (X)

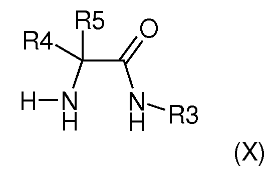

11. Process according to claim 10, wherein the open ring structure (X) is further reacted with a compound of formula R1-CO-R2.

12. Process according to claim 11, wherein the reaction with R1-CO-R2 takes place in a suitable solvent at a temperature comprised between 20°C and the reflux temperature of the solvent, for a time comprised between 1 h and 48 h.

13. Compounds of formula (I) as described in claims 1-5, for use in diagnosis and/or therapy.

14. Compounds of formula (I) as described in claim 13, for use as sodium channel inhibitors.

15. Compounds according to claim 14, for use as antiepileptics.

16. Pharmaceutical composition containing one or more compounds of formula (I) as described in claims 1-5.
